# EUROPÄISCHE PATENTSCHRIFT

(11) **EP 1 727 988 B1**
(45) Veröffentlichungstag und Bekanntmachung des Hinweises auf die Patenterteilung: **27.07.2011**
(21) Anmeldenummer: 05738776.3
(22) Anmeldetag: 17.03.2005
(51) Int. Cl.: F04D 29/04

(54) **PUMPE**
PUMP
POMPE

(30) Priorität: 18.03.2004 DE 102004013746; 18.03.2004 DE 102004013747; 20.04.2004 DE 102004019721
(43) Veröffentlichungstag der Anmeldung: 06.12.2006
(73) Patentinhaber: CircuLite, Inc., Saddle Brook, NJ 07663 (US)
(72) Erfinder: AKDIS, Mustafa, 52074 Aachen (DE); REUL, Helmut, verstorben (DE)
(74) Vertreter: Castell, Klaus
(86) Internationale Anmeldenummer: PCT/DE2005/000494
(87) Internationale Veröffentlichungsnummer: WO 2005/090791

(56) Entgegenhaltungen:
- WO-A-98/11650
- WO-A-02/066837
- US-A- 5 112 200
- US-A- 5 211 546
- US-B1- 6 234 772

## Beschreibung

Die Anmeldung betrifft eine Pumpe. Insbesondere betrifft die Erfindung eine Pumpe zur Förderung von Blut.

Aus dem Stand der Technik sind zahlreiche Pumpen mit Schaufelrädern bekannt, wobei die Schaufelräder meist mechanisch, mitunter auch lagerlos geführt sind. Beispielhaft für den medizintechnischen Bereich sei auf verschiedene Druckschriften hingewiesen:
So zeigt die US 6,116,862 eine Blutpumpe mit einem Flügelrad, welches in axialer Richtung kugelgelagert ist und in radialer Richtung durch einen sternförmigen Steg zentriert wird.

Die EP 0 904 117 B1 offenbart eine Blutpumpe, bei welcher ein Flügelrad axial ebenfalls über ein Kugellager fixiert ist. Die radiale Ausrichtung erfolgt über ein Gleitlager an einem Schaft eines Schaufelrads oder über Magnete, welche an einem Vorgehäuse installiert sind, das über einen Steg mit einem Hauptgehäuse der Pumpe verbunden ist.

In der DE 100 16 422 A1 ist eine Blutpumpe offenbart, welche ein Schaufelrad in einem Gehäuse fest installiert hat.

In der EP 0 599 138 A2 ist ein Schaufelrad einer Blutpumpe auf einer in ein separates Gehäuse ragenden Welle angeordnet.

Die US 5,840,070 zeigt einen Rotor an einem Schaft, wobei sowohl an Schaufelrädern des Motors als auch am Schaft eine Ausrichtung durch zahlreiche Magnete erfolgt.

Die DT 26 18 829 A1 zeigt eine mehrstufige Kreiselpumpe, bei welcher die einzelnen Pumpenstufen aus Kunststoff bestehen und durch axiale Vorspannung eine Verformung erfahren, bei welcher Seitenwände einzelner Stufengehäuse gegen ein Mantelrohr gepresst werden, um dort einen dichten Anschluss zu erreichen.

Die DT 298 21 565 U1 zeigt eine lagerlose Blutpumpe. Ein Antrieb eines Schaufelrads erfolgt über eine Magnetkupplung, die zu einer axialen Anziehung des Schaufelrads zum Motor hin führt. Das Schaufelrad ist in einem Pumpengehäuse innerhalb eines begrenzten Spiels frei beweglich und fördert bei Antrieb des Schaufelrads Blut in eine radiale Abströmrichtung. Um das axial zuströmende Blut um 90° zu einer radialen Abströmung umzulenken, übt das Schaufelrad einen Impuls auf das Blut aus. Damit das sich drehende Schaufelrad nicht in Folge des Gegenimpulses axial am Gehäuse aufsetzt, weisen die Schaufeln des Schaufelrads an ihrer von der Zuströmung abgewandten Seite Tragflächen auf. In Ruhezustand liegt das Schaufelrad auf dem Boden auf, bei beginnender Drehung hebt es langsam gegen die Durchtlussrichtung vom Boden ab. Sodann lagert sich das Schaufelrad axial über hydrodynamische Kräfte und radial über Magnetkräfte.

Die US 5,385,581 und die US 5 112 200 zeigen Blutpumpen mit reinen Magnetlagern, die ein Flügelrad mit einer elektromagnetischen Lagereinrichtung berührungslos lagern. Hierzu ist ein elektronisches Mess- und Regelsystem vorgesehen, welches in Folge der komplexen Lagerung zu einer voluminösen Bauform führt. Außerdem muss für eine aktive Schaufelradzentrierung Mehrenergie zugeführt werden.

Die US 5,211,546 zeigt eine Blutpumpe, bei der ein angetriebenes Flügelrad über Permanentmagnete verfügt und im Gehäuse zusammenwirkende elektrischen Wicklungen vorgegeben sind. Diese üben ein Antriebsdrehmoment aus und dienen gleichzeitig als Magnet-Axiallagerung. In Radialrichtung wird der Rotor durch ein oder mehrere hydrodynamische Radiallager stabilisiert.

Die Berlin Heart AG hat ein implantierbares Herzunterstützungssystem in Form einer Axialpumpe entwickelt. Auf dem Markt ist dieses unter dem Namen INCOR (eingetragene Marke) erhältlich. Ein Laufrad rotiert durch ein Magnetlager berührungsfrei schwebend und übernimmt mit Drehzahlen von bis zu 12.000 Umdrehungen pro Minute die eigentliche Pumpfunktion. Dies entspricht einem möglichen Blutfluss von 7 1/min gegen 150 mmHg. Ein dem Laufrad nachgeschaltetes Nachleitrad nimmt die Drehbewegung aus dem rotierenden Blut, baut dabei zusätzlich Druck auf und leitet das Blut zur Aorta weiter. Die Magnete der Axialpumpe sind für die berührungsfreie Lagerung des Laufrads mit einer Regelelektronik verbunden, um die Magnetfeldstärke im zeitlichen Verlauf schnell an eine geänderte Lage des Laufrads anpassen zu können. Die Pumpe hat eine elektrische Leistungsaufnahme von 8,5 W. Mit einem externen Akku wird eine Laufzeit von etwa 12 Stunden erreicht.

Die US 6,227,817 B1 schlägt eine Blutpumpe vor, bei der ein angetriebenes Flügelrad berührungslos über Magnetkräfte gelagert wird. Hierzu sind am Flügelrad Permanenunagnete vorgesehen, während am Gehäuse der Blutpumpe aktive Magnetspulen vorgesehen und mit einer regelbaren Spannungsquelle verbunden sind. Ein regelbares Magnetfeld hält das Flügelrad im Blutstrom.

Die EP 0 900 572 A1 zeigt eine Zentrifugalpumpe zum Fördern von Blut. Ein angetriebenes Flügelrad verfügt über Permanentmagnete, und im Gehäuse sind auch hier elektrische Wicklungen vorgesehen. Diese üben ein Antriebsdrehmoment und gleichzeitig eine einstellbare Querkraft auf das Flügelrad aus. Die Position des Flügelrads ist somit aktiv steuerbar.

Die US 5,470,208 B offenbart eine Pumpe, bei welcher ein Impeller elektromagnetisch angetrieben wird, wobei eine berührungslose Lagerung entweder über entgegengesetzt wirkende Magnetkräfte oder über entgegengesetzt wirkende Magnet- und Fluidkräfte bewirkt wird. Die vorgeschlagenen Kraftpaare lagern den Impeller sowohl in axialer als auch in radialer Richtung.

Die JP 2002 315824 A zeigt eine Blutpumpe, bei welcher ein Impeller axial über ein Kugel-Kalotten-System oder über hydrodynamische Kräfte gelagert ist. Die radiale Lagerung erfolgt über hydrodynamische Kräfte oder über Magnete.

Der Erfindung liegt die Aufgabe zu Grunde, eine besonders kostengünstige, energiesparende und verschleißarme Pumpe zu schaffen.

Im Hinblick auf die mögliche Verwendung in der Medizintechnik als Blutpumpe, beispielsweise als Mikrodiagonalblutpumpe, betrifft die Erfindung zudem weitere Aspekte: nach dem Stand der Technik ist es für Kreiselpumpen üblich, zur Lagerung des Schaufelrads mindestens ein verschleißbehaftetes mechanisches Gleitlager zu verwenden. Im Rahmen von invivo-Studien, bei welchen derartige Blutpumpen erprobt wurden, zeigte sich immer wieder die Problematik der Blutgerinnselbildung im Zuströmbereich in die Pumpe. An Stegen zur Fixierung des Schaufelrads an einer stromaufwärtigen Seite kam es bei den Untersuchungen mit Blut zu Thrombenablagerungen. In Bezug auf die medizintechnische Anwendung liegt der Erfindung die Aufgabe zu Grunde, eine Blutpumpe zur Verfügung zu stellen, bei welcher mit sehr kostengünstig herstellbaren Mitteln bei großer Zuverlässigkeit eine Thrombenablagerung im Zuströmbereich der Pumpe vermieden wird. Gleichzeitig soll die Pumpe verschleißfrei sein.

Die Aufgaben löst eine Blutpumpe mit einem Schaufelrad mit einer Drehachse in einem Pumpengehäuse und mit einem axialen Zuströmkanal zu dem Schaufelrad, wobei das Schaufelrad über ein Magnet-Axiallager und ein hydrodynamisches Radiallager im Betrieb zu dem Gehäuse berührungsfrei gelagert ist, wobei das Magnetaxiallager und das hydrodynami sche Radiallager in einer Ringhülse um das Schaufelrad kombiniert sind und das Magnetaxiallager separat vom Antrieb ausgestaltet ist. Erfindungsgemäß ist das Schaufelrad also gleichzeitig durch ein strömungsmechanisches Lager und ein Magnetlager so geführt, dass im Betrieb ein Aufsetzen oder Anschlagen des Schaufelrads an das Pumpengehäuse oder des Schaufelrads an andere stationäre Teile der Pumpe vermieden wird. Eine herausragende Eigenschaft dieser Hybridlagerung basiert auf der Möglichkeit, mit ausschließlich passiven Lagerelementen auszukommen, das heißt es sind keinerlei aktive Steuer- und Regelelemente wie Sensoren und Aktoren für die Funktionstüchtigkeit der Lagerung erforderlich.

Gegenüber der ebenfalls lagerlosen Blutpumpe aus der DE 298 21 565 U1 kennzeichnet sich die vorliegend vorgeschlagene Pumpe dadurch, dass die axiale Lagerung zumindest überwiegend durch Magnetkräfte erfolgt, während in der zitierten Gebrauchsmusterschrift die axiale Lagerung hydrodynamisch erfolgt. Die vorliegend vorgeschlagene Lösung bringt demgegenüber den großen Vorteil, dass ein axiales Anschlagen des Schaufelrads an das Pumpengehäuse auch bei Inbetriebnahme der Pumpe aus dem Stillstand sicher vermieden wird. In der DE 298 21 565 U1 hingegen liegt das Schaufelrad im Stillstand der Blutpumpe am Pumpengehäuse an. Bei Beginn der Rotation des Schaufelrads schaben deshalb die Schaufeln zunächst über den Gehäuseboden.

Die vorliegende Erfindung setzt die Erkenntnis um, dass ein axiales Anschlagen oder Schaben des Schaufelrads am Pumpengehäuse erhebliche Auswirkungen auf die Lebensdauer und die Betriebssicherheit der Pumpe haben kann. Daher wird vorliegend über ein Magnetlager eine axiale Sicherung des Schaufelrads erreicht, während eine radiale Stabilisierung zumindest auch durch ein strömungsmechanisches Lager bewirkt wird.

Bei der Erfindung ist eine Schaufelradlagerung besonders kostengünstig dadurch herstellbar, dass am Umfang des Schaufelrads ein kombiniertes Lager vorgesehen ist. Es ist von besonderem Vorteil, wenn das kombinierte Axial- und Radiallager die axiale Stabilisierung des Schaufelrads über Magnete und die radiale Stabilisierung des Schaufelrads über ein strömungsmechanisches Lager im Betrieb der Pumpe zur Verfügung stellt.

Zusätzlich zur Kosteneinsparung, die ein kombiniertes Lager ermöglicht, kann die Pumpe auch besonders klein bauen. Daher eignen sich beide vorgeschlagenen Pumpen besonders zum Einsatz in einer Kreiselblutpumpe, besonders als Blutpumpe zur Förderung von Blut.

Das passiv wirkende Magnet-Axiallager kann besonders einfach aus Permanentmagneten hergestellt sein. Beispielhaft wird hierfür die Verwendung des Werkstoffs Neodym-Eisen-Bor (NdFeB) vorgeschlagen. Insbesondere bieten sich für das Magnet-Axiallager zwei permanentmagnetische Ringe an, von denen vorteilhaft ein Statormagnet in ein Pumpengehäuse unbeweglich integriert sein kann, während ein Rotormagnet des Magnet-Axiallagers in eine Hülse um das Schaufelrad integriert und mit dessen Rotation verknüpft ist. Auf diese Weise können beide Permanentmagnete außerhalb der eigentlichen Strömungsbahnen gehalten werden, wobei dennoch ein geringer Abstand zwischen dem Statormagnet und dem Rotormagnet erreicht werden kann.

Unabhängig hiervon wird vorgeschlagen, dass das insbesondere magnetische Axiallager eine Kipprückstellkraft gegen ein Auskippen des Schaufelrads gegenüber einer Normalenebene zur Hauptachse ausübt. Das Axiallager eignet sich hierfür besonders, da ein Auskippen des Schaufelrads in der beschriebenen Weise stets auch eine am Umfang des Schaufelrads festzustellende axiale Verschiebung des Umfangs bewirkt. Wenn das Axiallager am Umfang des Schaufelrads jeweils lokal eine axiale Rückstellkraft bei Auslenkung bewirkt, bedeutet dies auch, dass dasselbe Magnetlager auch bei Kippbewegungen des Schaufelrads ein Kipprückstellmoment zur Verfügung stellt. Dieses Rückstellmoment kann bei gleichzeitigem Vorhandensein eines strömungsmechanischen Radiallagers in seiner Wirkung noch unterstützt werden. Eine solche Lagerung des Schaufelrads gegen axiale Verschiebungen und seitliche Verkippungen kann durch ein passiv wirkendes Permanentmagnetlager einfach bereitgestellt werden.

In einer bevorzugten Ausführungsform der vorgeschlagenen Pumpe mit Magnet-Axiallager sind ein Rotormagnet und ein Statormagnet des Magnet-Axiallagers in axialer Richtung magnetisiert, wobei der Statormagnet entgegengesetzt zum Rotormagneten magnetisiert ist. Insbesondere bei konzentrischer Anordnung der Ringmagnete resultiert hieraus eine anziehende Kraft zwischen den beiden Magnetringen, die sowohl in axialer als auch in radialer Richtung wirksam ist. Die axial anziehende Kraft dieser Konstellation bewirkt eine stabile Lagerung des Rotormagneten innerhalb des Statormagneten derart, dass das Schaufelrad sowohl gegen eine axiale Verschiebung als auch gegen Kippbewegungen stabilisiert ist. Die Stabilität der Lagerung und Kipprichtung basiert auf den anziehenden Rückstellkräften, die zwischen beiden Magnetringen wirksam sind und bestrebt sind, den Rotormagneten stets in die Mittellage des Statormagneten zurück zu versetzen.

Für einen sicheren und verschleißfreien Betrieb der Pumpe wird vorgeschlagen, dass die passive Magnet-Axiallagerung derart ausgelegt ist, dass die axial anziehenden Kräfte zwischen einem Rotormagneten und einem Statormagneten über zumindest den Großteil eines axialen Spiels des Schaufelrads stets größer sind als die in einer Magnetkupplung zwischen dem Schaufelrad und einem Antrieb wirkenden stromabwärts anziehenden Magnetkräfte. Eine solche Auslegung kennzeichnet sich dadurch, dass eine stromabwärtige axiale Auslenkung des Schaufelrads im beiderseitigen Magnetfeldeinfluss der Magnet-Axiallagerung und der Magnetkupplung eine resultierende Rückstellkraft stromaufwärts, also gegen die stromabwärtige Auslenkung, bewirkt. Die axial stromaufwärtige Rückstellkraft des Magnet-Axiallagers ist also größer als die axial stromabwärtige Auslenkkraft, die von der Magnetkupplung hervorgerufen wird. Hiermit ist gleichermaßen auch sichergestellt, dass die Kipprückstellkräfte der Magnetlagerung stets größer sind als die in der Magnetkupplung wirkenden Kräfte bei einer initialen Auslenkung des Schaufelrads. Die beschriebene Auslegung der Lager- und Kupplungsmagnete führt unmittelbar dazu, dass das Schaufelrad in axialer Richtung stets berührungslos gelagert ist. Dies entspricht unmittelbar einem Aspekt der Erfindung, denn das Schaufelrad hat auf diese Weise niemals axialen Kontakt mit dem stationären Pumpengehäuse oder dem Motordeckel. Die axiale Berührungsfreiheit tritt sowohl im Stillstand des Schaufelrads als auch im Betrieb der Pumpe ein. Jeglicher Verschleiß durch axialen Kontakt wird hiermit vermieden; vielmehr erfährt das Schaufelrad ein axiales Gleichgewicht im Einfluss der Magnetkupplung und dem Magnet-Axiallager axial stromabwärts des Statormagnets der Magnet-Axiallagerung, aber deutlich vor einem Anschlag des Schaufelrads an der stromabwärtigen Begrenzung seines Spiels durch das Gehäuse, also üblicherweise an die Magnetkupplung.

Es ist von Vorteil, wenn die axiale Rückstellwirkung auch bei einer axialen Auslenkung von bis zu 1 mm, bevorzugt von bis zu 3 mm, besonders bevorzugt von bis zu 5 mm, erfolgt.

Es sei erläutert, dass die zur Verdeutlichung einer relativen Position verwendeten Ausdrücke "stromaufwärts" und "stromabwärts" zwar vom Wortlaut einen unbedingten Bezug zu einer Betriebssituation der Pumpe herstellen. Diese relativen Positionsangaben sind jedoch auch außerhalb eines Betriebszustands der Pumpe zu identifizieren. So beziehen sich beide Angaben immer auf eine axiale Position bezüglich der Drehachse des Schaufelrads, wobei "stromaufwärts" näher an dem axialen Zuströmkanal zum Schaufelrad liegt als "stromabwärts".

Während ein Motordeckel bei bislang bekannten Pumpen stets eine Lagerfunktion ausübte - meist als Auflage für eine Kugellagerung, aber auch wie in der DE 298 21 565 U1 als strömungsmechanisches Axiallager - braucht der Motordeckel bei einer Pumpe nach der vorliegenden Erfindung lediglich als Trennung zwischen einer Motorkammer und einer Blutkammer zu dienen. Dies hat den Vorteil, dass der Motordeckel keinen nennenswerten mechanischen Belastungen ausgesetzt wird. Angesichts dessen und zur Vermeidung von Wirbelstromeffekten im Zwischenraum der Magnetkupplung und der damit verbundenen Erwärmung des Motordeckels wird vorgeschlagen, dass dieser aus einem nicht metallischen und/oder einem nicht magnetischen Werkstoff gefertigt ist, beispielsweise aus einem biokompatiblen Kunststoff oder Titanium.

Aus der Magnetlehre ist bekannt, dass ein Körper nicht allein anhand von passiven Magnetkräften räumlich stabil gehalten werden kann. Mindestens einer der sechs räumlichen Freiheitsgrade des Körpers verhält sich in einem passiven Magnetfeld stets instabil. Für den stabilen Schwebezustand des Körpers bedarf es immer aktiv geregelter Magnetkräfte oder zusätzlicher Lager, die den instabilen Freiheitsgrad ausschließen. Im Falle der vorgeschlagenen Magnet-Axiallagerung dergestalt, dass durch die passiven Magnetkräfte eine axiale Fixierung erfolgt, besteht der instabile Freiheitsgrad in der radialen Bewegungsfreiheit des Schaufelrads. Gemäß einer Erkenntnis der Erfindung bewirkt es jedoch keinen erhöhten Verschleiß, wenn das Schaufelrad im Stillstand eine radiale Berührung zum Gehäuse aufweist, solange diese im Betrieb aufgehoben wird. Beim Anlauf des Schaufelrads aus dem Stillstand treten gegenüber den axialen Kräften insbesondere durch die Umlenkung der Strömung nur äußerst geringe radiale Kräfte auf. Daher kann sich das anlaufende Schaufelrad auch praktisch unmittelbar von der radialen Berührung mit dem Gehäuse lösen und eine strömungsmechanisch bewirkte stabile Position in der Pumpe einnehmen.

Die Stabilisierung des Schaufelrads in radialer Richtung und somit das Einnehmen der vollständig berührungslosen Rotorlagerung im Betrieb kann bevorzugt durch Ausnutzen eines exzentrischen Ringspaltes um ein ausgelenktes Flügelrad erfolgen. Beim Vorsehen eines Magnet-Axiallagers mit zwei axial entgegengesetzt magnetisierten Ringen wird eine radiale Auslenkung des Schaufelrads auf Höhe der Magnet-Axiallagerung durch die ungleiche radiale Nähe zwischen Statormagnet und Rotormagnet noch verstärkt. Im Betrieb muss eine Sekundärströmung für ein strömungsmechanisches Radiallager deshalb so eingestellt werden, dass das strömungsmechanische Radiallager bei Auslenkung des Schaufelrads eine radiale Rückstellkraft auf das Schaufelrad ausübt, welche größer ist als die die Auslenkung weitertreibende Radialkraft des Magnet-Axiallagers. Das strömungsmechanische Radiallager kann in dieser Situation unterstützt werden von einer zusätzlichen Zentrierungswirkung einer Magnetkupplung.

Dabei ist alternativ und kumulativ darauf zu achten, dass eine Hauptströmung, eine Magnetkupplung und ein Magnet-Axiallager so aufeinander eingeregelt werden, dass das Schaufelrad ein stabiles axiales Gleichgewicht behält, auch wenn axial stromabwärtige Impulse auf das Schaufelrad zeitlich konstant oder schwankend einwirken, wobei axiale Bewegungen des Schaufelrads insbesondere auf ein Maß von wenigen Millimetern beschränkt sein können, ohne dass eine stabile Lagerung instabil wird

Ein strömungsmechanisches Radiallager ist sehr kostengünstig auszuführen, weil die Zentrierung des Schaufelrads bei geeigneter Konstruktion des strömungsmechanischen Radiallagers selbsttätig erfolgen kann. Dies geschieht, wenn bei Auslenkung des Schaufelrads in demjenigen Bereich, in welchem sich das Schaufelrad einer Gehäusewand nähert, eine die Auslenkkraft übersteigende Rückstellkraft auf das Schaufelrad ausgeübt wird, bis dieses wieder zentriert im Zulaufkanal steht. Die Rückstellkraft kann als Zentrierungskraft insbesondere an einem Umfang des Schaufelrads ausgeübt werden.

Zum Erzielen eines zuverlässigen strömungsmechanischen Lagereffekts ist es von Vorteil, wenn eine das Schaufelrad umgebende und an diesem fixierte Ringhülse innerhalb einer umgebenden Wandung des Pumpengehäuses vorgesehen ist. Eine an die Rotation des Schaufelrads gekoppelte Ringhülse, welche um das Schaufelrad herum verläuft, macht die Pumpe einer strömungsmechanischen Lagerung insbesondere stromaufwärts der Schaufel, besonders bevorzugt etwa auf axialer Höhe eines stromaufwärtigen Anfangs des Schaufelrads, besonders zugänglich. Durch die Rotation der Ringhülse bei Rotation des Schaufelrads wird zudem die Wahrscheinlichkeit von Strömungstotbereichen an der Ringhülse weitestgehend eliminiert. Außerdem kann ein strömungsmechanisches Lager besonders effektiv Rückstellkräfte am Umfang der Ringhülse ausüben.

Es sei darauf hingewiesen, dass im Rahmen dieser Anmeldung vorgeschlagene strömungsmechanische Lager auch den Spezialfall einer hydrodynamischen Lagerung und einer sonstigen Fluidfilmlagerung mit einschließen können.

Es wird vorgeschlagen, dass die Pumpe neben einem axialen und/oder diagonal-axialen Hauptströmungskanal durch das Schaufelrad und dem axialen Zuströmkanal zum Hauptströmungskanal des Schaufelrads einen Sekundärkanal aufweist, wobei der Sekundärkanal eine Speiseöffnung und eine Mündungsöffnung aufweist, von welchen die Mündungsöffnung zum Zuströmkanal orientiert ist. Der Sekundärkanal erzwingt bei geeigneter Konstruktion eine Sekundärströmung in einer solchen Stärke, dass die Strömung durch den Sekundärkanal für die strömungsmechanische Lagerung des Schaufelrads verwendet werden kann. Die Lagerströmung kann beim Verlauf im Sekundärkanal von der Hauptströmung getrennt geführt werden und insbesondere auch der Hauptströmung durch das Schaufelrad im Wesentlichen entgegengesetzt orientiert verlaufen. Dies ermöglicht eine automatische Zirkulation von Fluid durch das strömungsmechanische Radiallager, ohne dass von der Hauptströmung eine Abzweigung erfolgen muss.

Es wird vorgeschlagen, dass im Betrieb der Pumpe die Lagerströmung von einer Abströmung vom Schaufelrad gespeist wird. Unmittelbar beim Verlassen des Schaufelrads ist die Energiehöhe des gepumpten Fluids besonders hoch, sodass gegenüber einem stromaufwärtigen Punkt eine Druckdifferenz herrscht, welche zum Erzeugen der Sekundärströmung ausgenutzt werden kann.

Um das Energiegefälle bestmöglich auszunutzen, kann die Sekundärströmung in die Zuströmung vom Schaufelrad münden. Unmittelbar vor dem Schaufelrad ist die Energiehöhe des gepumpten Fluids am niedrigsten.

Es sei darauf hingewiesen, dass eine Pumpe mit einem Schaufelrad, insbesondere eine derartige Blutpumpe, mit einer Lagerströmung, welche im Betrieb einer Hauptströmung im Wesentlichen entgegengerichtet verläuft, auch unabhängig von sämtlichen übrigen Merkmalen der vorliegenden Erfindung in Kombination mit einem Magnet-Axiallager vorteilhaft ist. Gleiches gilt für eine Pumpe, insbesondere eine Blutpumpe, bei welcher im Betrieb eine Lagerströmung von einer Abströmung vom Schaufelrad gespeichert wird, sowie für eine Pumpe, insbesondere eine Blutpumpe, bei welcher im Betrieb eine Lagerströmung in eine Zuströmung zum Schaufelrad mündet.

In einer bevorzugten Ausführungsform ist ein Sekundärkanal flächig erstreckt und in einer Dimension senkrecht zu seiner Fläche mehr als 100 µm, bevorzugt etwa 300 bis 700 µm, besonders bevorzugt etwa 500 µm, breit. Klassische hydrodynamische Gleitlager fangen die auf einen Rotor wirkenden Kräfte durch eine in einem strömungsmechanischen Schmierfilm entstehende Druckkraft auf. Hierzu muss die Dicke des Schmierfilms derart klein sein, dass die viskosen Kräfte die Trägheitskräfte übertreffen.

Übliche Spaltbreiten liegen bei etwa 10 µm. Nur dann können die im Viskoseschmierfilm entstehenden Kräfte den auf den Rotor wirkenden äußeren Kräften das Gleichgewicht halten und das radiale Anschlagen des rotierenden Teils an das Gehäuse verhindern.

Solange eine Mischreibung verhindert wird, bleibt die Lagerung verschleißfrei. Der wesentliche Nachteil einer solchen rein hydrodynamischen Radiallagerung für die Anwendung in einer Blutpumpe ist jedoch, dass die geringe Breite des Schmierspalts die Scherspannungen und somit die Hämolyserate der Blutpumpe signifikant erhöht und somit einen blutschonenden Einsatz am Patienten einschränkt. Darüber hinaus liegt bei dermaßen kleinen Spaltbreiten auch eine erhöhte Gefahr von Strömungstotbereichen vor, sodass sogar die Gefahr der Thrombenablagerung im Lagerbereich erhöht wird, was bei Blutpumpen für den Langzeiteinsatz unbedingt zu vermeiden ist. Die vorliegende Erfindung kann jedoch auch bei einer strömungsmechanischen Lagerung mit deutlich größeren Spaltbreiten, insbesondere bei Spaltbreiten bis etwa 500 µm, genutzt werden. Dies senkt die Gefahren der Hämolyse und der Thrombogenität erheblich.

Bei aufwendigen Versuchen zur Strömungsführung hat sich zudem gezeigt, dass es von Vorteil ist, wenn im Betrieb die Lagerströmung überwiegend axial in einem tangential und axial erstreckten Sekundärkanal verläuft. Der Sekundärkanal kann insbesondere ein Ringspalt sein.

Es sei ausdrücklich darauf hingewiesen, dass eine Blutpumpe mit einer strömungsmechanischen Radiallagerströmung, welche überwiegend axial in einem tangential und axial erstreckten Sekundärkanal verläuft, auch für sich genommen in Kombination mit einem Magnet-Axiallager vorteilhaft ist, insbesondere wenn der Sekundärkanal ein Ringspalt um das Schaufelrad ist. Gleiches gilt für eine Blutpumpe mit einem Sekundärkanal für eine strömungsmechanische Lagerung, wobei der Sekundärkanal eine Spaltbreite von mehr als 100 µm, bevorzugt von etwa 300 µm bis 700 µm, besonders bevorzugt von etwa 500 µm, hat.

Alternativ und kumulativ zum vorgenannten wird vorgeschlagen, dass die Radiallagerströmung im Betrieb zwischen 5 % und 50 %, bevorzugt zwischen 10 % und 50 %, vor allem etwa 30 %, einer Hauptströmung ausmacht. Es hat sich gezeigt, dass bei einer Strömungseinstellung in diesem Spektrum ein ausreichendes strömungsmechanisches Radialpolster für das Schaufelrad erzeugt werden kann und Strömungsstagnation im Sekundärkanal sicher vermieden werden kann. Für eine Blutpumpe eignen sich besonders Volumenströme der Hauptströmung zwischen 1 1/min und 101/min, vor allem etwa 5 1/min.

Es wird vorgeschlagen, dass der Ringspalt so ausgeführt ist, dass bei radial zentriertem Schaufelrad keine radiale Berührung zwischen dem Schaufelrad und einer umgebenden Wandung des Pumpengehäuses auftritt. Unabhängig hiervon kann eine Mündungsöffnung des Sekundärkanals in axialer Richtung durch einen radialen Vorsprung vom Pumpengehäuse verdeckt sein. Vorteil eines umlaufenden Ringspalts um das Schaufelrad ist die verschleißfreie Lagerung, wobei ein Vorsprung des Gehäuses die Mündungsöffnung des Sekundärkanals genauso abdecken kann, dass trotz der umlaufenden Öffnung die Hauptströmung nicht in unkontrollierbarer Weise durch den Sekundärkanal strömt.

Zusätzlich zum strömungsmechanischen Radiallager kann eine mechanische radiale Spielbegrenzung für das Schaufelrad vorgesehen sein.

Für den Einsatz in Blutpumpen eignet sich besonders ein Sekundärkanal mit einer axialen Länge von 1 mm bis 20 mm, bevorzugt von etwa 5 mm. Für die Dimensionen des Schaufelrads wird ein Durchmesser von 2 mm bis 100 mm, bevorzugt von 15 mm bis 25 mm, besonders bevorzugt von etwa 20 mm, vorgeschlagen. Unabhängig hiervon wird vorgeschlagen, dass das Schaufelrad mit einer Umdrehungszahl von weniger als 50.000 U/min angetrieben wird, bevorzugt zwischen etwa 2.000 und 10.000 U/min, insbesondere mit etwa 5.000 U/min.

Unabhängig hiervon wird vorgeschlagen, dass eine Einrichtung zum Erhöhen der Strömungsenergie durch einen Hauptströmungskanal der Pumpe strömenden Fluids zwischen einer Speiseöffnung und einer Mündungsöffnung des Sekundärkanals vorgesehen ist. Auf diese Weise kann ein deutliches Druckgefälle zwischen Speisung und Mündung des Sekundärkanals erzeugt werden, was die Strömungsrichtung des Fluids im Sekundärkanal sicher vorhersagbar macht. Die Einrichtung zum Erhöhen der Strömungsenergie kann insbesondere das angetriebene, rotierende Schaufelrad selbst sein.

Der Zentrierungseffekt durch die radialen Rückstellkräfte am strömungsmechanischen Radiallager kann durch eine Magnetkupplung zwischen einem Motor und dem Flügelrad unterstützt werden. Eine axial anziehende Stirndrehkupplung zwischen Schaufelrad und Motor weist bei radialer Auslenkung eine Steifigkeit auf, welche bestrebt ist, die Kupplungsmagneten am Schaufelrad gegenüber den Magneten am Motor radial zu zentrieren. Das gleichzeitige Einwirken der radialen Rückstellkräfte zum einen des strömungsmechanischen Radiallagers und zum anderen der Magnetkupplung trägt zur höheren Gesamtstabilität und Laufruhe des Schaufelrads bei.

Durch die Ausnutzung dieses Effekts im Leckagebereich der Pumpe kann die radiale Stabilisierung des Rotors ohne jegliche mechanische Gleitlager erreicht werden. Die Ausnutzung der ohnehin vorhandenen unvermeidbaren inneren Verluste der Pumpe zur Gewährleistung der funktionellen Sicherheit macht den erfinderischen Vorschlag besonders ökonomisch. Dieser ökonomische Aspekt wird durch die Ausnutzung von ohnehin vorhandenen Radialkräften in einer Magnetkupplung noch stärker hervorgehoben. Dabei kann die Ausführung äußerst kompakt sein, insbesondere wenn ein Rotormagnet und ein Statormagnet des Magnet-Axiallagers zumindest einen Teil eines strömungsmechanischen Radiallagers räumlich einschließen.

Der Vollständigkeit halber sei darauf hingewiesen, dass die vorliegende Erfindung an einem strömungsmechanischen Radiallager auch mit schmalen Lagerkanälen gemäß der klassischen hydrodynamischen Schmiertheorie anstandslos funktioniert. Für eine Blutpumpe ist jedoch eine größere Spaltbreite, insbesondere bis zu 500 µm, vorzuziehen. Insofern stellt die Pumpe gemäß der vorliegenden Erfindung eine besonders einfache, betriebssichere und kostengünstige Lösung für eine sehr blutschonende Rotorlagerung unter der Möglichkeit des Ersetzens aller mechanischen Lager durch berührungslose Lager zur Verfügung.

Bei einer Pumpe mit einem Magnet-Axiallager und einer Magnetkupplung zwischen Schaufelrad und Motor nimmt das Schaufelrad im Betrieb bei konstanter Zuströmung und geeigneter Einstellung der Magnete und der Kanalgeometrie ein axiales Gleichgewicht zwischen einem unbeeinflussten Ruhezustand am Magnet-Axiallager und einem anderen unbeeinflussten Ruhezustand an der Magnetkupplung ein. Bei einer zeitlichen Änderung der Zuströmung, beispielsweise in Folge eines Druckstoßes bei einem Herzschlag in der medizintechnischen Anwendung der Pumpe, baut sich stromaufwärts der Pumpe kurzzeitig eine von der quasi-statischen Kraft der Hauptströmung unterschiedliche Temporärkraft auf. Die Kraftdifferenz stört das axiale Gleichgewicht des Schaufelrads, sodass das Schaufelrad während der Dauer der Kraftänderung eine andere axiale Lage einnimmt. Der berührungslose Schwebezustand des Schaufelrads kann während des Betriebs der Pumpe auch Auslenkungen unterworfen sein, wobei sowohl die axialen als auch die radialen Auslenkungen proportional zu den auf das Schaufelrad einwirkenden Kräften sind. Bei geeigneter Einstellung bewegt sich das Schaufelrad im Betrieb der Pumpe daher je nach Drehzahl, Druck- und Strömungsbedingungen sowie äußeren Kräften, beispielsweise infolge eines Sturzes des Patienten, innerhalb des Pumpengehäuses frei im Fördermedium, ohne dabei in mechanischen Kontakt mit dem Pumpengehäuse zu kommen.

Die Bewegung des Schaufelrads in axialer Richtung ist ein Maß für die auf den Rotor einwirkenden Strömungskräfte, die aus dem Druckverlauf des strömenden Fluids am Schaufelrad resultieren. Daher wird alternativ und kumulativ zum Vorgenannten vorgeschlagen, dass die Pumpe einen Sensor zum größenmäßigen Erfassen der axialen Verschiebung des Schaufelrads aufweist.

Der Sensor kann die axialen Bewegungen des Schaufelrads insbesondere ohne jeglichen Kontakt, also nicht-invasiv, erfassen und somit Rückschlüsse über den Arbeitspunkt der Pumpe liefern. Die über den Sensor gemessene Verschiebungsstrecke ist proportional der Druckverteilung am Rotor und kann in Verbindung mit dem hydraulischen Kennfeld der Pumpe mit dem Pumpenfluss korreliert werden. Für eine nicht-invasive Messung liegt der Sensor bevorzugt außerhalb des Kanalnetzes, insbesondere durch eine Abschottung vom Kanalnetz getrennt. Der Sensor kann beispielsweise kapazitiv, induktiv oder resistiv die Verschiebung des Schaufelrads ermitteln. Daher erübrigt sich auch ein direkter Zugang zum Kanalnetz.

In einer bevorzugten Ausführungsform ist eine Auswertungseinrichtung vorgesehen, welche aus den Messwerten des Sensors in Kombination mit den bekannten Werten für Drehzahl, Spannung und Strom den Druck und/oder die Strömungsgeschwindigkeit, insbesondere die Durchflussrate und im Falle der medizintechnischen Anwendung beispielsweise die Herzfrequenz innerhalb der Pumpe berechnet und numerisch oder alphanumerisch anzeigt. Aus den erfassten Sensorsignalen sind Anomalien der Herzfrequenz wie beispielsweise Extrasystolen oder Kammerflimmern zu erkennen.

Es ist von Vorteil, wenn das auf diese Weise erfasste Signal des Sensors als Eingangsgröße für die Regelung der Pumpe entsprechend den physiologischen Anforderungen eingesetzt wird. Es kann auch zu diagnostischen Zwecken beim Patienten eingesetzt werden.

Vorteilhaft kann bei Verwendung eines Sensors auf separate Druck- und Flusssensoren, welche in Blutkontakt stehen und beim Langzeiteinsatz driftbehaftet sind, vollständig verzichtet werden. Die gesamte Blutpumpe wird damit einerseits im Hinblick auf den Langzeiteinsatz bei einem Patienten betriebssicherer und gleichzeitig einfacher im Handling und in der Überwachung der Pumpfunktionen.

Die Erfindung wird nachstehend anhand eines Ausführungsbeispiels unter Bezugnahme auf die Zeichnung weiter erläutert. Hierin zeigen
- Figur 1: in einem Längsschnitt eine Blutpumpe mit einer kombinierten Magnet-Axiallagerung und strömungsmechanischen Radiallagerung eines Flügelrads und mit einem Sensor,
- Figur 2: die Blutpumpe aus Figur 1 in einer teilgeschnittenen Ansicht gemäß Kennzeichnung II- II in Figur 1 und
- Figur 3: die Blutpumpe aus Figur 1 und 2 in einer ausschnittweisen Vergrößerung des Längs- schnitts aus Figur 1 mit einer schematischen Kennzeichnung von Strombahnen.

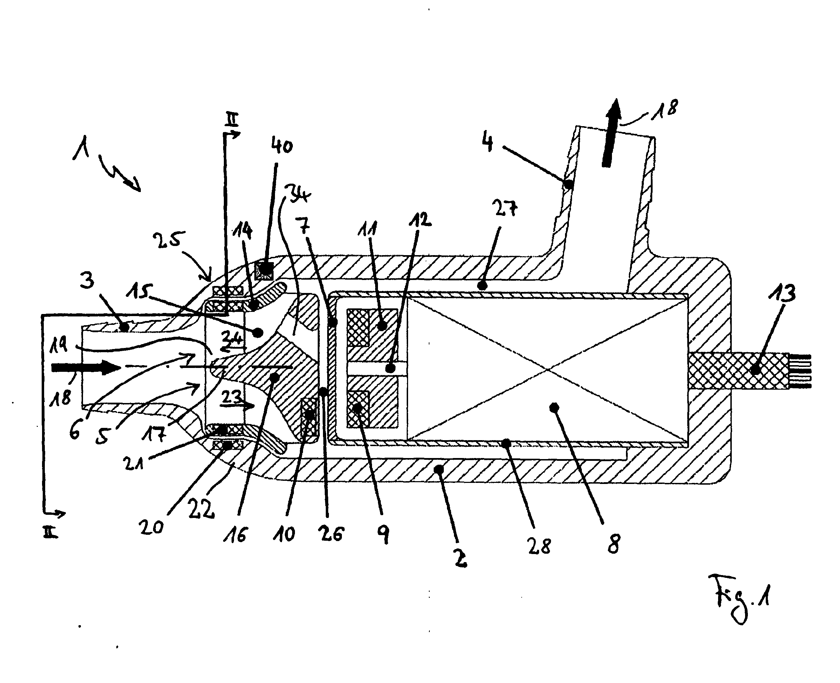

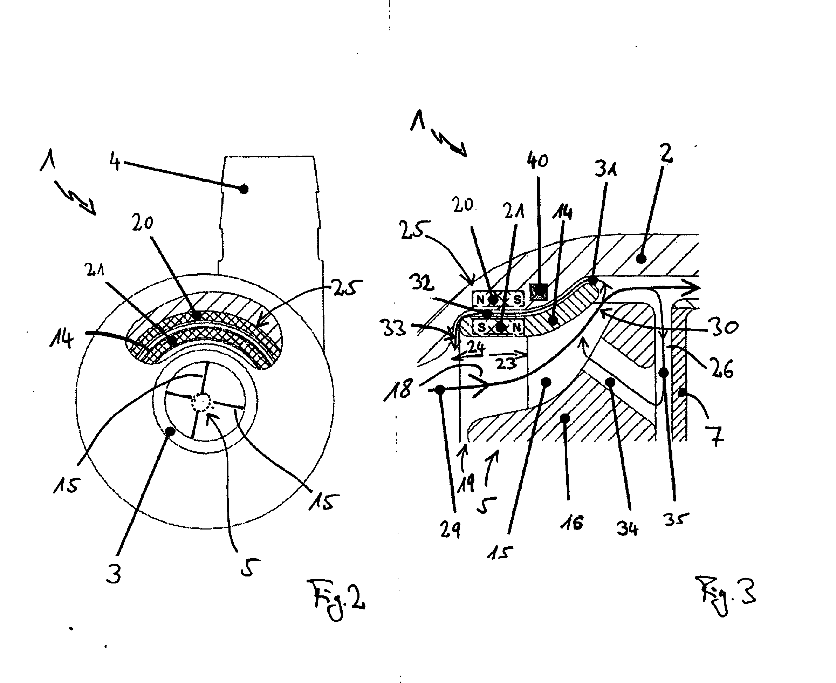

Die Blutpumpe 1 in den Figuren 1, 2 und 3 besteht im Wesentlichen aus einem Pumpengehäuse 2 mit einem Zulaufkanal 3 und einem Auslaufstutzen 4. Im Inneren des Pumpengehäuses 2 liegen ein Schaufelrad 5 in einem hierfür vorgesehen Raum 6 und hinter einem Motordeckel 7 ein Motor 8 zum Antrieb des Schaufelrads 5 über eine Magnetkupplung 9, 10. Die motorseitigen Kupplungsmagneten 9 liegen in einem Polschuh 11, welcher auf einer Welle 12 des Motors angeordnet ist. Zum Antrieb des Motors 8 verläuft zu diesem eine Kabelleitung 13.

An einem Umfang des Flügelrads 5 ist eine Ringhülse 14 vorgesehen. Die Ringhülse 14 ist mit Schaufeln 15 eines zentralen Schaufelkörpers 16 des Schaufelrads 5 verbunden, sodass eine Rotation des zentralen Schaufelkörpers 16 um eine Drehachse 17 zugleich eine ebensolche Rotation der Ringhülse 14 um die Drehachse 17 bewirkt. An einem bezüglich einer vorgesehen Durchströmungsrichtung 18 stromaufwärtigen Ende 19 des Schaufelrads 5 auf dessen axialer Höhe angeordnet liegen zwei Permanentmagnetringe 20, 21. Ein Statormagnet 20 ist in eine Wandung 22 des Pumpengehäuses 2 integriert; konzentrisch mit dem Statormagneten 20 und in axialer Erstreckung identisch ist ein Rotormagnet 21 in die Ringhülse 14 des Schaufelrads 5 integriert. Der Stator 20 und der Rotor 21 sind in axialer Richtung magnetisiert, wobei der Stator entgegensetzt zum Rotor magnetisiert ist (Kennzeichnung der Magnetpole durch "N" bzw. "S"). Es sei darauf hingewiesen, dass auch in ihrer axialen Erstreckung unterschiedliche Längen von Stator- und Rotormagnet eingesetzt werden können. Auf diese Weise kann der Stabilitätsbereich der Lagerung erweitert werden.

Die entgegengerichtet orientierten Magnetringe Stator 20 und Rotor 21 fungieren als Magnet-Axiallager. Die Kupplungs-Magneten 9, 10 ziehen sich gegenseitig an, sodass auf das Schaufelrad 5 eine Kraft ausgeübt wird, welche das Schaufelrad 5 zum Motordeckel 7 hin beschleunigt. Gleichzeitig würde eine solche Verschiebung aber auch den Rotor 21 gegenüber dem Stator 20 in einer axialen Auslenkrichtung 23 verschieben. Hierdurch werden der S-Pol des Rotors vom N-Pol des Stators sowie der N-Pol des Rotors vom S-Pol des Stators entfernt und gleichzeitig der S-Pol des Rotors dem S-Pol des Stators näher gebracht. Infolge dessen üben die Permanentmagnetringe Stator 20 und Rotor 21 eine Kraft in eine axiale Rückstellrichtung 24 aus. Die axiale Rückstellrichtung 24 ist entgegengerichtet der Auslenkung 23 durch die Kupplungs-Magnete 9, 10. Insofern wirken die Permanentinagnetringe Stator 20 und Rotor 21 als Magnet-Axiallager 25 gegen eine axiale Verschiebung des Schaufelrads 5.

Die Rückstellkraft 24 wird mit zunehmender Verschiebung des Rotors 21 gegenüber dem Stator 20 zunehmend größer, bis die Rückstellkraft ein Maximum dort erreicht, wo die beiden S-Pole des Stators 20 und des Rotors 21 untereinander zum liegen kommen. Ein stromabwärtiger Lagerspalt 26 ist jedoch so schmal bemessen, dass die beiden S-Pole des Rotors 21 und des Stators 20 nicht untereinander zum Liegen kommen können. Somit kann das magnetische Axiallager 25 auch durch mechanische Einwirkungen auf das Flügelrad 5 nicht aus seiner stabilen Lage gebracht werden.

In Folge der Anziehungskraft zwischen den Kupplungs-Magneten 9, 10 nimmt das Flügelrad 5 jedoch eine Gleichgewichtslage (in der Zeichnung nicht dargestellt) ein, bei welcher das Flügelrad 5 gegenüber einer unbeeinflussten Ursprungslage (in der Zeichnung dargestellt) des Magnet-Axiallagers 25 eine Verschiebung 23 einnimmt und behält. Die axiale Gleichgewichtslage liegt weiter entfernt vom Zulaufkanal 13 als in den Figuren dargestellt, also in der Wortwahl der vorliegenden Anmeldung stromabwärts.

Während das Schaufelrad 5 axial berührungsfrei innerhalb des Gehäuses 2 liegt, liegt die Ringhülse 14 im Stillstand der Pumpe 1 seitlich an der Wandung 22 des Gehäuses 2 an, da der Stator 20 und der Rotor 21 nicht zusätzlich zur axialen magnetischen berührungslosen Lagerung eine stabile Lagerung in radialer Richtung erreichen können. Vielmehr wird das Schaufelrad 5 radial ausgelenkt, bis es durch Kontakt der Ringhülse an der Gehäusewandung 22 anliegt. Dieser radial ausmittige Zustand wird durch die ungleiche Beabstandung des Rotors 21 vom Stator 20 über den Umfang des Magnet-Axiallagers mechanisch begrenzt. Im Bereich der Magnet-Kupplung 9, 10 zwischen dem Schaufelrad 5 und dem Motor 8 liegt das Schaufelrad 5 nicht mehr am Gehäuse 2 an, da sich dieses ausgangs der Ringhülse 14 zu einem ringförmigen Durchströmkanal 27 aufweitet. Der Durchströmkanal 27 führt um eine Motorwand 28 hin zum Abflussstutzen 4.

Durch die radial stabile Lage des Schaufelrads 5 an der Magnet-Kupplung 9, 10 bei gleichzeitig stabil ausmittiger Lage des Schaufelrads 5 an der Magnet-Axialkupplung 25 ist das Schaufelrad 5 gegenüber der Drehachse 17 - zusätzlich zu einer leichten Parallelverschiebung - leicht ausgelenkt. Auch dies bewirkt eine leichte Verrückung der Pole zwischen Stator 20 und Rotor 21, sodass vom Magnet-Axiallager 25 bereits im stabil ausmittigen Zustand eine leichte Rückstellkraft auf das Schaufelrad 5 ausgeübt wird. Infolge dessen reicht eine nur geringe zusätzliche Zentrierungskraft, um die Ringhülse 14 des Schaufelrades 5 wieder berührungsfrei innerhalb der Gehäusewandung 22 zentrieren zu können.

Im Betrieb der Pumpe treibt der Motor 8 über die Magnetkupplung 9, 10 das Schaufelrad 5 zu einer Rotation um die Drehachse 17 an. Zuströmendes Blut fließt in einer Hauptströmung 29 von etwa 5 l/min durch das Schaufelrad 5 zum Hauptdurchströmkanal 27 und durch den Auslassstutzen 4 aus der Blutpumpe 1 heraus. Innerhalb des Schaufelrads 5 wird das Blut auf dem zentralen Schaufelkörper 16 nach außen umgelenkt und im Verlauf über die Schaufeln 15 nach außen beschleunigt, sodass es an einer Abströmung 30 vom Schaufelrad dieses in diagonaler Richtung und mit besonders großer Energiehöhe verlässt. Durch eine Speiseöffnung 31 zwischen Ringhülse 14 und Gehäusewandung 22 an der Abströmung 30 vom Schaufelrad 5 gelangt Blut in einer Lagerströmung 32 wieder stromaufwärts zu einer Mündungsöffnung 33 des Lagerkanals 32 und von dort wieder in die Zuströmung 29 zum Schaufelrad 5. Die Druckdifferenz zwischen der Speiseöffnung 31 und der Mündungsöffnung 33 des Lagerkanals 32 ist so groß, dass etwa zwei Fünftel und somit etwa 2 l/min vom Blutfluss als Leckageströmung abgezweigt werden und somit zwischen der Ringhülse 14 und der Gehäusewandung 22 im Wesentlichen axial rückströmen. Dabei entsteht ein strömungsmechanisches Radiallager im Lagerspalt 32 und somit auch zwischen dem Magnet-Axiallager 25. Das Magnet-Axiallager 25 ist in Kombination mit dem strömungsmechanischen Radiallager stromaufwärts der Schaufeln 15 angeordnet, um eine besondere gute Hebelwirkung für Rückstellkräfte zu haben.

Durch die Umlenkung der Blutströmung innerhalb des Schaufelrads 5 übt das Blut eine Ausstellkraft entlang der Verschiebungsrichtung 24 auf das Schaufelrad aus. Das Magnet-Axiallager ist jedoch so stark dimensioniert, dass sowohl die Kupplungs-Anziehungskraft 9, 10 in Richtung 23 als auch die durch die Strömung erzeugte Ausstellkraft in Richtung 24 die Axial-Lagerkraft zwischen den Magnetringen 20, 21 nicht aufwiegt. Daher stellt sich auch bei Durchströmung des Schaufelrads 5 mit Blut eine axial berührungsfreie Gleichgewichtslage des Schaufelrads 5 innerhalb des Gehäuses 2 ein. Diese Gleichgewichtslage im Betrieb ist gegenüber der Gleichgewichtslage im Stillstand des Schaufelrads 5 lediglich um ein bestimmtes Maß stromaufwärts oder stromabwärts verschoben (in der Zeichnung nicht dargestellt). Bei einer Schwankung der Zuströmung 29 erfährt das Schaufelrad 5 eine kurzzeitige axiale und mit der Strömungsschwankung korrespondierende Auslenkung; sie bleibt aber bei sämtlichen in der Verwendung als Blutpumpe zu erwartenden Druck- und Strömungszuständen axial berührungsfrei zum Gehäuse 2 und dem Motordeckel 7.

Durch das strömungsmechanische Radiallager 32 entfernt sich die Ringhülse 14 beim Anlaufen des Schaufelrads 5 unmittelbar von der Gehäusewandung 22 und nimmt eine stabile, zum Gehäuse 2 vollständig berührungslose Lage auch in radialer Hinsicht ein. In dieser Situation ist das Schaufelrad 5 im Pumpengehäuse 2 durch das Magnet-Axiallager 25 und durch das strömungsmechanische Radiallager 32 ohne Berührung zwischen dem Schaufelrad 5 und dem Gehäuse 2 stabil in seiner Rotation gelagert. Am Umfang des Schaufelrads 5, vorliegend am Umfang der Ringhülse 14, stabilisiert demzufolge ein kombiniertes Axial-(25) und Radiallager 32 das Schaufelrad 5. Eine Blutschädigung und Thrombenbildung wird sicher vermieden, weil das strömungsmechanische Gleitlager 32 eine Spaltbreite von etwa 500 µm hat und es nirgends zu Strömungstotbereichen kommt. Hierfür sorgen unter anderem auch Spülbohrungen 34 und eine hierdurch erzwungene Spülströmung 35 im zentralen Schaufelkörper 16.

In der Wandung 22 des Gehäuses 2 ist zusätzlich ein Sensor 40 vorgesehen, welcher eine radiale und axiale Verschiebung des Schaufelrads 5 hochfein registriert, wenn sich das Schaufelrad 5 im Gehäuse 2 axial und/oder radial verschiebt. Über das Sensorsignal des Sensors 40 lässt sich die exakte Lage des Schaufelrads 5 im Gehäuse 2 bestimmen. Dies erlaubt einen unmittelbaren Rückschluss auf die Strömungsverhältnisse des Bluts durch die Blutpumpe 1 und somit auf den Betriebszustand der Pumpe.So können beispielsweise die Herzfrequenz eines Patienten und Herzschlaganomalien durch infolge äußerer Einwirkungen auf den Patienten einwirkende Kräfte anhand von Messwerten des Sensors 40 erkannt werden.

## Patentansprüche

1. Blutpumpe (1) mit einem Schaufelrad (5) mit einer Drehachse (17) in einem Pumpengehäuse (2, 3) und mit einem axialen Zuströmkanal (8) zu dem Schaufelrad (5), wobei das Schaufelrad (5) über ein Magnet-Axiallager (25) und ein hydrodynamisches Radiallager (14, 32) im Betrieb zu dem Gehäuse (2, 3) berührungsfrei gelagert ist, ***dadurch gekennzeichnet, dass*** ein Rotormagnet (21) des Magnet-Axiallagers und das hydrodynamische Radiallager (14) in einer Ringhülse (14) um das Schaufelrad (5) kombiniert sind und die Ringhülse (14) separat vom Antrieb ausgestaltet ist.

2. Pumpe nach einem der vorhergehenden Ansprüche, ***dadurch gekennzeichnet, dass*** das Axiallager eine Kipprückstellkraft gegen ein Auskippen des Schaufelrads gegenüber einer Normalenebene zur Hauptachse ausübt, wobei bevorzugt ein Rotormagnet und ein Statormagnet eines Magnet-Axiallagers in axialer Richtung magnetisiert sind, wobei eine Ausrichtung entgegengesetzt ist.

3. Pumpe nach einem der vorhergehenden Ansprüche, ***dadurch gekennzeichnet, dass*** eine axial stromabwärtige Auslenkung des Schaufelrads, ausgehend von einem axialen Gleichgewicht, eine gegen die Auslenkung wirkende resultierende Rückstellkraft bewirkt, insbesondere soweit die axiale Auslenkung 1 mm, bevorzugt 3 mm, besonders bevorzugt 5 mm, nicht übersteigt.

4. Pumpe nach einem der vorhergehenden Ansprüche, ***dadurch gekennzeichnet, dass*** das Schaufelrad im Stillstand und im Betrieb der Pumpe axial berührungslos zum Pumpengehäuse ist

5. Pumpe nach einem der vorhergehenden Ansprüche, ***dadurch gekennzeichnet, dass*** das Schaufelrad im Stillstand radial am Pumpengehäuse anliegt, während es im Betrieb der Pumpe radial zum Pumpengehäuse berührungslos ist.

6. Pumpe nach einem der vorhergehenden Ansprüche, ***dadurch gekennzeichnet, dass*** die Pumpe einen axialen und/oder diagonal-axialen Hauptströmungskanal durch das Schaufelrad und einen axialen Zuströmkanal zu dem Schaufelrad und zusätzlich einen Sekundärkanal mit einer Speiseöffnung und einer Mündungsöffnung aufweist, wobei die Mündungsöffnung zum Zuströmkanal orientiert ist und wobei im Betrieb eine Lagerströmung einer Hauptströmung im Wesentlichen entgegengerichtet orientiert verläuft, wobei der Sekundärkanal bevorzugt ein Ringspalt um das Schaufelrad ist.

7. Pumpe nach einem der vorhergehenden Ansprüche, ***dadurch gekennzeichnet, dass*** eine Mündungsöffnung eines Sekundärkanals in axialer Richtung durch einen radialen Vorsprung eines Pumpengehäuses verdeckt ist.

8. Pumpe nach einem der vorhergehenden Ansprüche, ***dadurch gekennzeichnet, dass*** ein Magnet-Axiallager stromaufwärts von Schaufeln des Schaufelrads angeordnet ist.

9. Pumpe nach einem der vorhergehenden Ansprüche, **gekennzeichnet durch** eine Auswertungseinrichtung zum Berechnen bzw. Erkennen von Druck, Durchflussrate, Strömungsschwankungsfrequenz und/oder Strömungsschwankungsanomalien sowie von äußeren Belastungen auf den Patienten anhand von Messwerten eines Sensors.

## Claims

1. A blood pump (1) with a paddle wheel (5) with a rotational axis (17) in a pump casing (2, 3) and with an axial inflow channel (8) toward the paddle wheel (5), the paddle wheel (5) in operation being mounted without contact with the casing (2, 3) via a magnetic axial heating (25) and a hydrodynamic radial bearing (14, 32), ***characterized in that*** a rotor magnet (21) of the magnetic axial hearing (5) and the hydrodynamic radial bearing (14) are combined in a ring sleeve (14) around the paddle wheel (5) and the ring sleeve (14) is formed separately from the drive.

2. The pump according to one of the afore-mentioned claims, ***characterized in that*** the axial bearing exerts a tilt restoring force against a tilting of the paddle wheel vis-à-vis a normal plane relative to the main axis, a rotor magnet and a stator magnet of a magnetic axial bearing being preferably magnetized in the axial direction, and have an opposed orientation.

3. The pump according to one of the afore-mentioned claims, ***characterized in that*** an axially downstream deflection of the paddle wheel from an axial balance causes a restoring force which acts against the deflection, more specifically insofar as the axial deflection does not exceed 1mm, preferably 3mm, most preferably 5mm.

4. The pump according to one of the afore-mentioned claims, ***characterised in that*** during standstill and during operation, the paddle wheel is axially contact free relative to the pump casing.

5. The pump according to one of the afore-mentioned claims, ***characterized in that*** during standstill the puddle wheel rests radially against the pump casing white being radially contact free relative to the pump casing during operation.

6. The pump according to one of the afore-mentioned claim, ***characterized in that*** the pump has an axial and/or diagonally axial main flow channel through the paddle wheel and an axial inflow channel toward the paddle wheel and additionally a secondary channel with a feed aperture and an outlet aperture, the outlet aperture being oriented toward the inflow channel and a bearing flow running substantially in an opposite orientation to the main flow during operation, the secondary channel preferably being an annular gap around the paddle wheel.

7. The pump according to one of the afore-mentioned claims, ***characterized in that*** an outlet aperture of a secondary channel is covered in an axial direction by a radial protrusion of a pump casing.

8. The pump according to one of the afore-mentioned claims, ***characterized in that*** a magnetic axial bearing is disposed upstream of paddles of the paddle wheel.

9. The pump according to one of the afore-mentioned claims, ***characterized by*** a processing device for calculating, respectively detecting pressure, flow rate, flow fluctuation frequency and/or flow fluctuation anomalies as well as outer stresses on the patient by means of values measured by a sensor.

## Revendications

1. Pompe a sang (1) avec une roue à aubes (5) avec un axe de rotation (17) dans un boîtier de pompe (2, 3) et avec un canal d'afflux (X) axial vers la roue a aubes (5), la roue à aubes (5) étant montée via un palier magnétique axial (25) et un palier hydrodynamique radial (14, 32) sans entrer en contact avec le boîtier (2, 3) durant, le fonctionnement, ***caractérisée en ce qu'***un aimant de rotor (21) du palier magnétique axial (5) et le palier hydrodynamique radial (14) sont combinés dans un manchon annulaire (14) autour de la roue à aubes (5) et que le manchon annulaire (14) est séparé de l'entraînement.

2. Pompe selon l'une quelconque des revendications précédentes, ***caractérisée en ce que*** le palier axial exerce une force de rétablissement contre un basculement de la roue à aubes par rapport à un plan normal vis-à-vis de l'axe principal, un aimant de rotor et un aimant de stator d'un palier magnétique axial étant magnétisés de préférence dans une direction axiale, leur orientation étant opposée.

3. Pompe selon l'une quelconque des revendications précédentes, *carartérisée en ce qu'*une déviation de la roue à aube axialement vers l'aval, à partir d'un équilibre axial, provoque une force de rétablissement s'exerçant contre la déviation, notamment dans la mesure où la déviation axiale est de 1 mm, de préférence de 3 mm, et avec une préférence particulière de 5mm.

4. Pompe selon l'une quelconque des revendications précédentes, ***caractérisée en ce qu'***à l'arrêt et durant le fonctionnement, la roue à aubes n'est pas en contact axial avec le boîtier de la pompe.

5. Pompe selon l'une quelconque des revendications précédentes, ***caractérisée en ce qu'***à l'arrêt, la roue à aubes est en appui radial sur le boîtier de la pompe, alors qu'elle n'est, pas en contact radial avec le boîtier de la pompe durant le fonctionnement.

6. Pompe selon l'une quelconque des revendications précédentes, ***caractérisée en ce que*** la pompe comporte un canal d'écoulement principal axial et/ou diagonalement axial à travers la roue à aubes et un canal d'afflux vers la roue à aubes et un canal secondaire additionnel avec une ouverture d'approvisionnement et une embouchure, l'embouchure étant orientée vers le canal d'afflux et un courant de palier étant essentiellement orienté dans la direction inverse par rapport à un courant principal, le canal secondaire étant de préférence un espace annulaire autour de la roue à aubes.

7. Pompe selon l'une quelconque des revendications précédentes, *carartérisée en ce qu*'une embouchure d'un canal secondaire est couverte dans la direction axiale par une saillie radiale d'un boîtier de pompe.

8. Pompe selon l'une quelconque des revendications précédentes, ***caractérisée en ce qu'***un palier magnétique axial est disposé en amont d'aubes de la roue à aubes.

9. Pompe selon l'une quelconque des revendications précédentes, ***caractérisée par*** un dispositif de traitement pour calculer, respectivement détecter la pression, le débit, la fréquence des fluctuations du courant et/ou les anomalies de fluctuation du courant ainsi que des agressions externes de l'organisme du patient à partir de valeurs mesurées à l'aide d'un capteur.
